# EUROPEAN PATENT APPLICATION

(11) **EP 3 662 878 A1**
(43) Date of publication of application: **10.06.2020**
(21) Application number: 18841454.4
(22) Date of filing: 01.08.2018
(51) Int. Cl.: A61F 13/15, A61F 13/534, A61F 13/535

(54) **METHOD FOR MANUFACTURING ABSORBENT BODY, DEVICE FOR MANUFACTURING ABSORBENT BODY, AND ABSORBENT BODY**

(30) Priority: 04.08.2017 JP 2017151314; 01.09.2017 JP 2017168869
(71) Applicant: Zuiko Corporation, Osaka 566-0045 (JP)
(72) Inventor: NAKAMURA, Hideyuki, Settu-Shi Osaka 566-0045 (JP); FUJITA, Yukihiko, Settu-Shi Osaka 566-0045 (JP); KOSHIJIMA, Miwa, Settu-Shi Osaka 566-0045 (JP)
(74) Representative: Dr. Gassner & Partner mbB
(86) International application number: PCT/JP2018/028900
(87) International publication number: WO 2019/026971

(57) **Abstract**

The objective of the present invention is to manufacture an absorbent body with which it is easy to enhance liquid permeability and liquid diffusion.
(i) A belt-like main sheet (10) in which a non-woven fabric sheet (14) constitutes at least part of a first main surface (10s) is conveyed in a longitudinal direction of the main sheet (10); (ii) a slit (16) is formed in the non-woven fabric sheet (14) at a first position on a conveying path in such a way as to form an incision (17) in the first main surface (10s); (iii) at a second position, particulate matter (11) capable of absorbing and retaining liquid is dispersed into a region of the first main surface (10s) that includes the incision (17), in such a way that the particulate matter (11) is held in the non-woven fabric sheet (14); and (iv) at a third position, a covering sheet (18) which prevents passage of the particulate matter (11) is overlaid on the region of the first main surface (10s) in which the particulate matter (11) has been dispersed. At the second position, the non-woven fabric sheet (14) includes a bulky structure (14k) which constitutes at least part of the first main surface (10s) and which has voids allowing the particulate matter (11) to enter.

## Description

### [Technical Field]

The present invention relates to a method for manufacturing an absorbent body, a device for manufacturing the absorbent body and the absorbent body, and more specifically, to a method for manufacturing an absorbent body where particulate matter is held in a non-woven fabric sheet, a device for manufacturing the absorbent body and the absorbent body.

### [Background Art]

An absorbent body where particulate matter capable of absorbing and retaining liquid is held in a non-woven fabric sheet is used, for example, for disposable diapers, disposable shorts and women's sanitary products. Such an absorbent body is used in a state of an individual piece that is cut and separated from a continuous body.

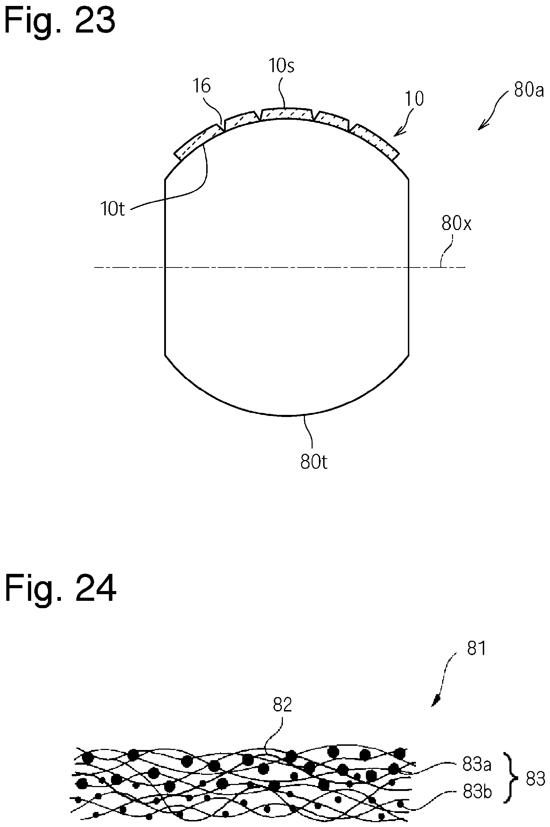

For example, Fig. 24 is a schematic view showing the structure of an absorbent body 81. As shown in Fig. 24, in the absorbent body 81, particles 83a and 83b of particulate matter 83 having different particle diameters are held in voids between fabrics of a web 82 (for example, see Patent Literature 1).

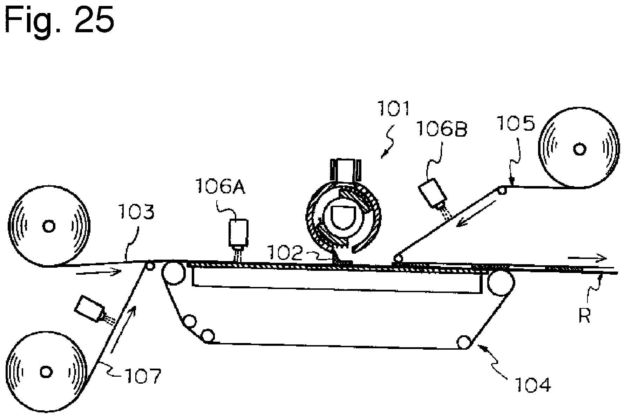

Fig. 25 is a schematic view showing the structure of a device for manufacturing a continuous body R of the absorbent body. As shown in Fig. 25, this device disperses particulate matter 102 in a predetermined pattern from a dispersing device 101 to one main surface of a first sheet 103 being conveyed by a conveyer 104, and pastes a second sheet 105 thereonto. To the pasted surfaces of the first sheet 103 and the second sheet 105, a fixing agent is applied by fixing agent dispersing devices 106A and 106B, thereby enhancing the fixability of the particulate matter 102. Moreover, before the particulate matter 102 is dispersed to one main surface of the first sheet 103, a third sheet 107 is pasted to the other main surface of the first sheet 103 (for example, see Patent Literature 2).

### [Citation List]

### [Patent Literatures]

[Patent Literature 1] JP 2006-305327 A
[Patent Literature 2] JP 2002-178429 A

### [Summary of the Invention]

### [Problem to be Solved by the Invention]

As the first sheet 103 where the particulate matter 102 is dispersed, a bulky non-woven fabric sheet may be used. In this case, the particulate matter is disposed and held in spaces between fabrics of the non-woven fabric sheet.

However, if the fiber density of the non-woven fabric sheet is made a fiber density such that the particulate matter can be held in a predetermined dispersed state, when liquid is supplied to the surface of the absorbent body, the liquid is substantially uniformly and gradually diffused in the surface direction of the non-woven fabric sheet and absorbed and retained inside the non-woven fabric sheet, so that it is not easy to enhance liquid passage (liquid permeability) and liquid diffusion (liquid diffusibility) for the non-woven fabric sheet.

In view of such circumstances, the present invention is intended to provide a method for manufacturing an absorbent body by which an absorbent body with which it is easy to enhance liquid permeability and liquid diffusibility can be manufactured, a device for manufacturing the absorbent body and the absorbent body.

### [Means for Solving the Problem]

To solve the above-mentioned problem, the present invention provides a method for manufacturing an absorbent body structured as follows:

A method for manufacturing an absorbent body is provided with : (i) a first step of conveying a belt-like main sheet in a longitudinal direction of the main sheet along a predetermined conveying path, the main sheet having a first and second main surfaces, the main sheet including a non-woven fabric sheet that is made of a non-woven fabric and constitutes at least part of the first main surface; (ii) a second step of forming, at a first position on the conveying path, a slit on the non-woven fabric sheet of the main sheet being conveyed so as to form an incision in the first main surface; (iii) a third step of dispersing, at a second position on the conveying path on a downstream side of the first position in a conveying direction of the main sheet, particulate matter capable of absorbing and retaining liquid into a region, including the incision, of the first main surface of the main sheet being conveyed, so as to cause the particulate matter to be held in the non-woven fabric sheet; and (iv) a fourth step of overlaying, at a third position on the conveying path on the downstream side of the second position in the conveying direction of the main sheet, a covering sheet on the region, in which the particulate matter has been dispersed, of the first main surface of the main sheet being conveyed, the covering sheet preventing passage of the particulate matter. At the second position, the non-woven fabric sheet of the main sheet includes a bulky structure constituting at least part of the first main surface of the main sheet and having a void allowing the particulate matter to enter.

In the above-described method, the main sheet may be constituted by only the non-woven fabric sheet or may include other than the non-woven fabric sheet. The first main surface of the main sheet may be constituted by only the non-woven fabric sheet or may be constituted by the non-woven fabric sheet and other than the non-woven fabric sheet.

According to the above-described method, the particulate matter dispersed to the first main surface of the main sheet at the third step enters the non-woven fabric sheet also from the cut surfaces of the slit as well as the first main surface and becomes tangled with the fibers of the non-woven fabric sheet to be held in the void between the fibers of the non-woven fabric sheet. For this reason, the amount of particulate matter held in the non-woven fabric sheet can be easily increased compared with when no slit is formed.

Moreover, with the absorbent body where the slit is formed in the non-woven fabric sheet, liquid of the same amount can be easily made to be absorbed and retained by the particulate matter in a wider range in a shorter time compared with when no slit is formed.

Consequently, the absorbent body with which it is easy to enhance liquid permeability and liquid diffusibility can be manufactured.

The slit may be formed in an arbitrary direction. For example, the slit may also be formed in a direction orthogonal to the conveying direction of the main sheet or in a direction intersecting the conveying direction.

Preferably, at the second step, the slit is formed so as to cause the slit to extend in the longitudinal direction of the main sheet. The slit may extend continuously or extend discontinuously or intermittently.

In this case, the slit can be easily formed by a simple method such as applying a blade to the main sheet being conveyed and cutting it.

While it is possible to form a slit passing between the first main surface and the second main surface of the main sheet, when this is done, there are cases where the particulate matter dispersed to the first main surface of the main sheet passes through the slit to be away from the main sheet and is not held in the non-woven fabric sheet.

Preferably, at the second step, the slit is formed only from the first main surface up to in front of the second main surface of the main sheet.

In this case, it is possible to make all the dispersed particulate matter held in the non-woven fabric sheet, and the amount of particulate matter held in the non-woven fabric sheet can be increased. Moreover, since the conveyance of the main sheet is easy, it is easy to increase the manufacturing capacity of the absorbent body by increasing the conveying speed of the main sheet.

Preferably, at the second step, when perspectively viewed vertically to the first main surface of the main sheet, the slit is formed only in the region, of the first main surface of the main sheet, in which the particulate matter is to be dispersed at the third step.

In this case, in the post-process during manufacture, the possibility can be reduced that the particulate matter having entered the slit moves along the slit and spills out from the end portions of the divisional piece.

The main sheet may be such that the non-woven fabric sheet of the main sheet previously includes the bulky structure by using, for example, a fiber-raised non-woven fabric, a non-woven fabric using crimped fibers, an air through non-woven fabric that is made multi-layered. In this case, at the first step, the main sheet where the non-woven fabric sheet includes the bulky structure is conveyed. The bulky structure may be formed while the main sheet is being conveyed as follows:

Preferably, the method for manufacturing the absorbent body is further provided with a fifth step of forming, at a fourth position on the conveying path on an upstream side of the second position in the conveying direction of the main sheet, the bulky structure on the non-woven fabric sheet of the main sheet being conveyed. At the third step, the particulate matter enters the void of the bulky structure formed at the fifth step and is held therein.

In this case, since the amount of particulate matter held in the non-woven fabric sheet can be increased by forming the fiber-raised part in a fiber-raised state suitable for holding the particulate matter before the particulate matter is dispersed, it is easy to enhance liquid permeability and liquid diffusibility.

Moreover, to solve the above-mentioned problem, the present invention provides a device for manufacturing an absorbent body structured as follows:

A device for manufacturing an absorbent body is provided with: (a) a conveying device that conveys a belt-like main sheet in a longitudinal direction of the main sheet along a predetermined conveying path, the main sheet having a first and second main surfaces, the main sheet including a non-woven fabric sheet that is made of a non-woven fabric and constitutes at least part of the first main surface; (b) a slit forming device that is disposed so as to adjoin the first position on the conveying path and forms a slit on the non-woven fabric sheet of the main sheet being conveyed by the conveying device so as to form an incision in the first main surface; (c) a dispersing device that is disposed so as to adjoin a second position on the conveying path on a downstream side of the first position in a conveying direction of the main sheet and disperses particulate matter capable of absorbing and retaining liquid into a region, including the incision, of the first main surface of the main sheet being conveyed by the conveying device, so as to cause the particulate matter to be held in the non-woven fabric sheet; and (d) a covering device that is disposed so as to adjoin a third position on the conveying path on the downstream side of the second position in the conveying direction of the main sheet and overlays a covering sheet on the region, in which the particulate matter has been dispersed, of the first main surface of the main sheet being conveyed by the conveying device, the covering sheet preventing passage of the particulate matter. At the second position, the non-woven fabric sheet of the main sheet includes a bulky structure constituting at least part of the first main surface of the main sheet and having a void allowing the particulate matter to enter.

In the above-described structure, the main sheet may be only the non-woven fabric sheet or may include other than the non-woven fabric sheet. The first main surface of the main sheet may be constituted by only the non-woven fabric sheet or may be constituted by the non-woven fabric sheet and other than the non-woven fabric sheet.

According to the above-described structure, the particulate matter dispersed to the first main surface of the main sheet enters the non-woven fabric sheet also from the cut surfaces of the slit as well as the first main surface and becomes tangled with the fibers of the non-woven fabric sheet to be held in the void between the fibers of the non-woven fabric sheet. For this reason, the amount of particulate matter held in the non-woven fabric sheet can be easily increased compared with when no slit is formed.

Moreover, with the absorbent body where the slit is formed in the non-woven fabric sheet, liquid of the same amount can be easily made to be absorbed and retained by the particulate matter in a wider range in a shorter time compared with when no slit is formed.

Consequently, the absorbent body with which it is easy to enhance liquid permeability and liquid diffusibility can be manufactured.

The slit may be formed in an arbitrary direction. For example, the slit may also be formed in a direction orthogonal to the conveying direction of the continuous body or in a direction intersecting the conveying direction.

Preferably, the slit forming device is structured so as to form the slit in such a way that the slit extends in the longitudinal direction of the main sheet. The slit may extend continuously or extend discontinuously or intermittently.

In a preferred mode, the slit forming device is provided with a tool having a blade. The blade is disposed so as to enter the conveying path at the first position on the conveying path from the first main surface side of the main sheet being conveyed along the conveying path. The blade forms the slit on the non-woven fabric sheet of the main sheet being conveyed along the conveying path so as to cause the slit to extend in the longitudinal direction of the main sheet. The slit may extend continuously or extend discontinuously or intermittently.

In this case, a slit extending in the longitudinal direction can be formed with a simple structure.

While it is possible to form a slit passing between the first main surface and the second main surface of the main sheet, when this is done, there are cases where the particulate matter dispersed to the first main surface of the main sheet passes through the slit to be away from the main sheet and is not held in the non-woven fabric sheet.

Preferably, the slit forming device is structured so as to form the slit only from the first main surface up to in front of the second main surface of the main sheet.

In a preferred mode, the slit forming device is provided with a tool having a blade. The blade is disposed so as to enter the conveying path only up to in front of the second main surface of the main sheet being conveyed along the conveying path, from the first main surface side of the main sheet being conveyed along the conveying path, at the first position on the conveying path. The blade forms the slit only from the first main surface up to in front of the second main surface of the main sheet being conveyed along the conveying path.

In this case, it is possible to make all the dispersed particulate matter held in the non-woven fabric sheet, and the amount of particulate matter held in the non-woven fabric sheet can be increased. Moreover, since the conveyance of the main sheet is easy, it is easy to increase the manufacturing capacity of the absorbent body by increasing the conveying speed of the main sheet.

Preferably, when perspectively viewed vertically to the first main surface of the main sheet, the slit forming device forms the slit only in a dispersion planned region, of the first main surface of the main sheet, in which the particulate matter is to be dispersed by the dispersing device.

In this case, in the post-process during manufacture, the possibility can be reduced that the particulate matter having entered the slit moves along the slit and spills out from the end portions of the divisional piece.

The main sheet may be such that the non-woven fabric sheet of the main sheet previously includes the bulky structure by using, for example, a fiber-raised non-woven fabric, a non-woven fabric using crimped fibers, an air through non-woven fabric that is made multi-layered. In this case, the conveying device conveys the main sheet where the non-woven fabric sheet includes the bulky structure. The bulky structure may be formed while the main sheet is being conveyed as follows:

Preferably, the device for manufacturing the absorbent body is further provided with a fiber raising device that is disposed so as to adjoin a fourth position on the conveying path on an upstream side of the second position in the conveying direction of the main sheet and forms the bulky structure on the non-woven fabric sheet of the main sheet being conveyed by the conveying device. The particulate matter dispersed by the dispersing device enters the void of the bulky structure formed by the fiber raising device and is held therein.

In this case, since the amount of particulate matter held in the non-woven fabric sheet can be increased by forming the fiber-raised part in a fiber-raised state suitable for holding the particulate matter before the particulate matter is dispersed, it is easy to enhance liquid permeability and liquid diffusibility.

Moreover, to solve the above-mentioned problem, the present invention provides an absorbent body structured as follows:

The absorbent body is provided with: (a) a main sheet having a first and second main surfaces, the main sheet including a non-woven fabric sheet that is made of a non-woven fabric and constitutes at least part of the first main surface, wherein an incision is formed in the first main surface and a slit communicating with the incision is formed in the non-woven fabric sheet; (b) particulate matter that is held in a void between fibers of the non-woven fabric sheet and is capable of absorbing and retaining liquid; and (c) a covering sheet that is overlaid on the first main surface and prevents passage of the particulate matter.

In the above-described structure, the main sheet may be constituted by only the non-woven fabric sheet or may include other than the non-woven fabric sheet. The first main surface of the main sheet may be constituted by only the non-woven fabric sheet or may be constituted by the non-woven fabric sheet and other than the non-woven fabric sheet.

According to the above-described structure, the amount of particulate matter held in the non-woven fabric sheet can be easily increased compared with when no slit is formed. Moreover, liquid of the same amount can be easily made to be absorbed and retained by the particulate matter in a wider range in a shorter time compared with when no slit is formed.

Consequently, the absorbent body with which it is easy to enhance liquid permeability and liquid diffusibility can be manufactured.

Preferably, the slit is formed only from the first main surface up to in front of the second main surface of the main sheet.

In this case, the amount of particulate matter held in the non-woven fabric sheet can be increased. Moreover, it is easy to increase the manufacturing capacity of the absorbent body.

Preferably, when perspectively viewed through vertically to the first main surface of the main surface, the slit is formed only in a dispersion region in which the particulate matter has been dispersed.

In this case, in the post-process during manufacture, the possibility can be reduced that the particulate matter having entered the slit moves along the slit and spills out from the end portions of the divisional piece.

### [Effects of the Invention]

According to the present invention, an absorbent body with which it is easy to enhance liquid permeability and liquid diffusibility can be manufactured.

### [Brief Description of the Drawings]

[Fig. 1] Fig. 1 is a schematic view showing the structure of an absorbent body manufacturing device (first embodiment).
[Figs. 2A to 2E] Figs. 2A to 2E are cross-sectional views showing an absorbent body manufacturing method (first embodiment).
[Fig. 3] Fig. 3 is a relevant part plan view of a slit forming device (first embodiment).
[Fig. 4] Fig. 4 is a plan view of a main sheet (first embodiment).
[Fig. 5] Fig. 5 is a cross-sectional view of the main sheet (first embodiment).
[Fig. 6] Fig. 6 is a schematic view showing a relevant part structure of an absorbent body manufacturing device (first modification).
[Figs. 7A and 7B] Figs. 7A and 7B are cross-sectional views of a main sheet (first modification).
[Fig. 8] Fig. 8 is a schematic view showing a relevant part structure of an absorbent body manufacturing device (second modification).
[Fig. 9] Fig. 9 is a schematic view showing a relevant part structure of an absorbent body manufacturing device (third modification).
[Fig. 10] Fig. 10 is a perspective view of the main sheet (fourth modification).
[Fig. 11] Fig. 11 is a perspective view of an absorbent body piece (fourth modification).
[Fig. 12] Fig. 12 is a perspective view of the main sheet (fifth modification).
[Fig. 13] Fig. 13 is a cross-sectional view of the main sheet (sixth modification).
[Figs. 14A to 14D] Figs. 14A to 14D are cross-sectional views of the main sheet (seventh modification).
[Figs. 15A to 15D] Figs. 15A to 15D are cross-sectional views of a main sheet and absorbent bodies (eighth modification).
[Fig. 16] Fig. 16 is a cross-sectional view of an absorbent body (ninth modification).
[Figs. 17A and 17B] Figs. 17A and 17B are cross-sectional views of a main sheet and an absorbent body (tenth modification).
[Figs. 18A and 18B] Figs. 18A and 18B are cross-sectional views of a main sheet and an absorbent body (tenth modification).
[Figs. 19A and 19B] Figs. 19A and 19B are cross-sectional views of a main sheet and an absorbent body (tenth modification).
[Figs. 20A and 20B] Figs. 20A and 20B are schematic views showing relevant part structures of absorbent body manufacturing devices (eleventh modification).
[Figs. 21A and 21B] Fig. 21A is a schematic view showing a relevant part structure of an absorbent body manufacturing device, and Fig. 21B is a schematic cross-sectional view (twelfth modification).
[Figs. 22A and 22B] Figs. 22A and 22B are enlarged cross-sectional views of the main sheet (twelfth embodiment).
[Fig. 23] Fig. 23 is a schematic cross-sectional view similar to Fig. 21B (twelfth modification).
[Fig. 24] Fig. 24 is a schematic view showing the structure of the absorbent body (first conventional example).
[Fig. 25] Fig. 25 is a schematic view showing the device for manufacturing the absorbent body (second conventional example).

### [Mode for Carrying out the Invention]

Hereinafter, an embodiment of the present invention will be described with reference to the drawings.

### <First embodiment>

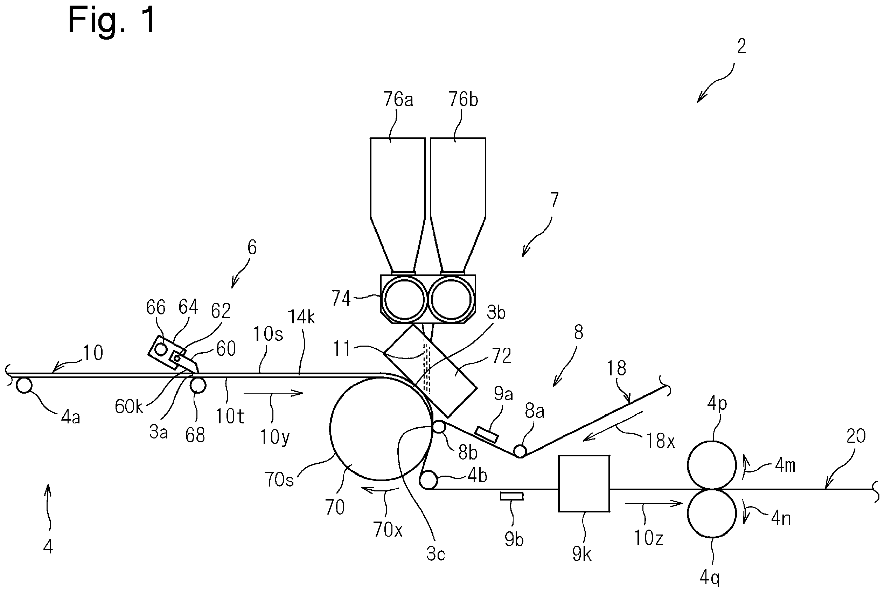

An absorbent body manufacturing method, an absorbent body manufacturing device and an absorbent body of a first embodiment will be described with reference to Figs. 1 to 5.

Fig. 1 is a schematic view showing the structure of an absorbent body manufacturing device 2. As shown in Fig. 1, the absorbent body manufacturing device 2 manufactures an absorbent body 20 as a continuous body while conveying a belt-like main sheet 10 in the direction indicated by the arrows 10y and 10z along a predetermined conveying path.

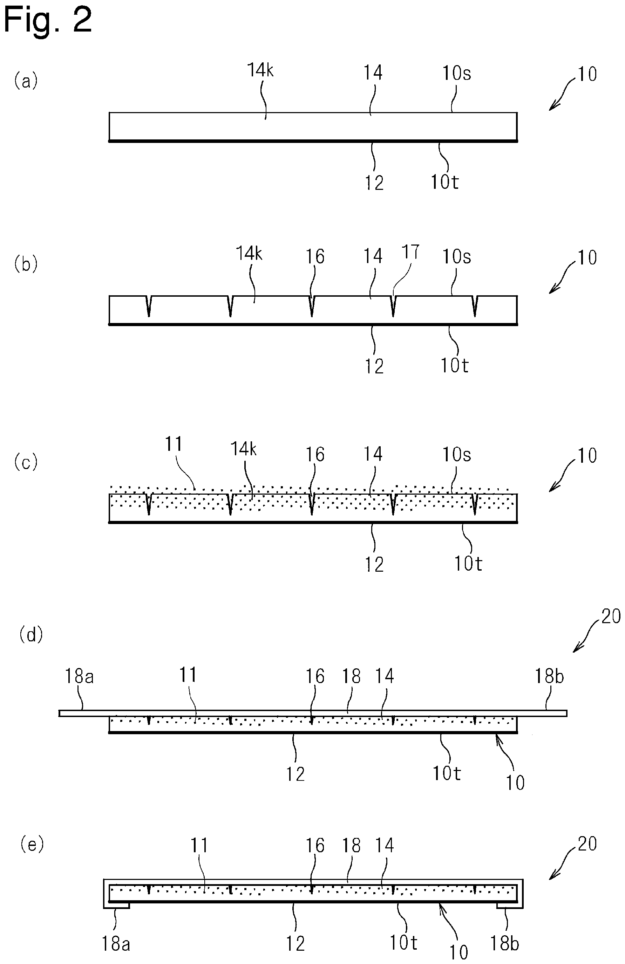

The main sheet 10 has a first and second main surfaces 10s and 10t, includes a non-woven fabric sheet 14 made of a non-woven fabric (see Figs. 2A to 2E), has at least part of the first main surface 10s constituted by the non-woven fabric sheet 14, and is conveyed in the longitudinal direction of the main sheet 10 by a conveying device 4. In the order from the upstream side toward the downstream side in the conveying direction of the main sheet 10, a slit forming device 6, a dispersing device 7 and a covering device 8 are disposed so as to adjoin a first to third positions 3a, 3b and 3c on the conveying path of the main sheet 10.

The slit forming device 6 forms a slit in the main sheet 10. The dispersing device 7 disperses particulate matter 11 to the first main surface 10s of the main sheet 10. The covering device 8 overlays a covering sheet 18 on the first main surface 10s of the main sheet 10 where the particulate matter 11 has been dispersed.

Figs. 2A to 2E are cross-sectional views of the main sheet 10 which is cut in the width direction (that is, a direction vertical to the longitudinal direction and thickness direction of the main sheet 10), and shows the manufacturing steps of the absorbent body 20. As shown in Fig. 2A, the main sheet 10 is a composite sheet where the non-woven fabric sheet 14 made of a non-woven fabric is pasted to one main surface of a support sheet 12, the first main surface 10s of the main sheet 10 is constituted by the non-woven fabric sheet 14, and the second main surface 10t of the main sheet 10 is constituted by the support sheet 12. The non-woven fabric sheet 14 previously includes a bulky structure 14k constituting at least part of the first main surface 10s of the main sheet 10 and having voids allowing the particulate matter 11 to enter.

For the non-woven fabric sheet 14 of the main sheet 10, an appropriate non-woven fabric is used; for example, an air-through treated non-woven fabric, that is, a non-woven fabric formed by arranging short fibers and applying hot air thereto is used. Here, the short fibers are fibers whose fiber length measured by the average fiber length measuring method (C method) of JIS L 1015 is less than 100 mm, preferably, less than 80 mm, further preferably, 70 mm. For the non-woven fabric sheet 14 of the main sheet 10, for example, a non-woven fabric constituted by short fibers with an average fiber length of approximately 50 mm is used.

For the non-woven fabric sheet 14 of the main sheet 10, a non-woven fabric using crimped fibers or an air through non-woven fabric that is made multi-layered may be used.

The support sheet 12 is formed of one layer or a plurality of layers. For the main sheet 10, the support sheet 12 and the non-woven fabric sheet 14 may be integrally formed, or the support sheet 12 and the non-woven fabric sheet 14 which are separate members may be integrally formed by bonding by hot melt or the like, heat welding, or the like.

For the support sheet 12, a sheet is used that is small in thickness and high in density compared with the non-woven fabric sheet 14. For the support sheet 12, a sheet is used that is high in diffusibility and capable of allowing liquid to penetrate in a wide area.

The main sheet 10 may be constituted by only the non-woven fabric sheet 14. Moreover, as in a tenth modification described later, the support sheet 12 of the main sheet 10 may include a covering sheet.

As shown in Fig. 1, the conveying device 4 includes guide rolls 4a and 4b disposed along the conveying path of the main sheet 10 and a pair of press contact rolls 4p and 4q rotating in the directions shown by the arrows 4m and 4n and sandwiching the main sheet 10 and the covering sheet 18. The conveying device 4 conveys, for example, the main sheet 10 wound off from an original fabric roll.

In the slit forming device 6, a tool 60 having a single-edged blade 60k which is a linear blade is disposed on one side with respect to the conveying path of the main sheet 10, that is, on the side opposed to the first main surface 10s of the main sheet 10, and a receiving roll 68 is disposed so as to be opposed to the end of the tool 60 on the other side, that is, on the side opposed to the second main surface 10t of the main sheet 10.

Although it is preferable to provide the receiving roll 68 because the main sheet 10 can be firmly supported and a slit can be easily formed in the non-woven fabric sheet 14 of the main sheet 10, a structure having no receiving roll 68 may be adopted.

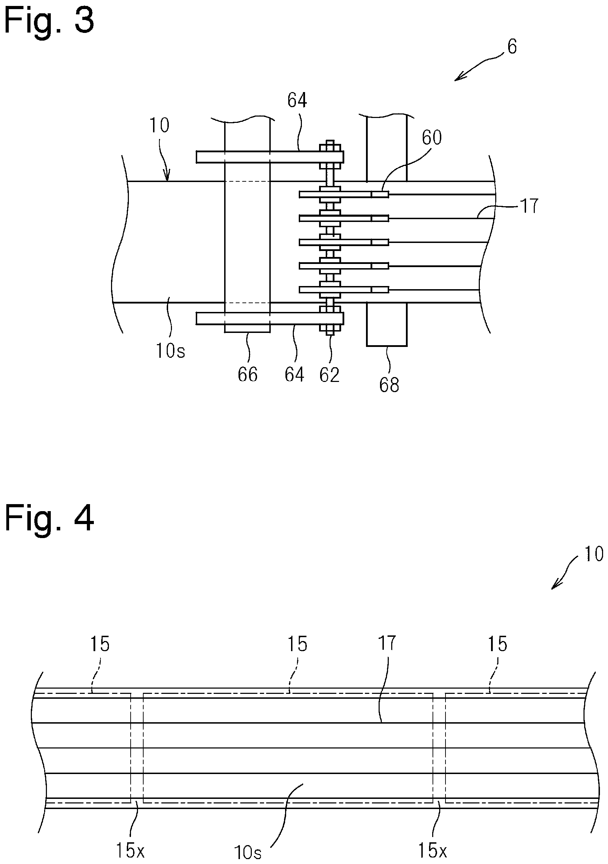

Fig. 3 is a relevant part plan view of the slit forming device 6. As shown in Fig. 3, a plurality of tools 60 are disposed at intervals. Although the number of tools 60 may be one or more, the provision of more than one tool increases the number of slits to enhance liquid permeability, liquid diffusibility and the holding amount of the particulate matter. The tools 60 may be disposed either at regular intervals or at irregular intervals. The intervals may be arbitrarily set. For example, the tools 60 are disposed with a pitch of 10 mm.

As shown in Figs. 1 and 3, the tools 60 are fixed to a tool support shaft 62, and are disposed parallel to the conveying direction (longitudinal direction) of the main sheet 10. The tool support shaft 62 is fixed to a support column 66 fixed to a non-illustrated base of the absorbent body manufacturing device 2, through a pair of attachment plates 64. With this structure, since the attachment, replacement and adjustment of the tools 60 are easy, it is easy to increase or decrease the number of attached tools 60 and change the attachment position according to the width and use of the absorbent body 20.

The single-edged blades 60k of the tools 60 are disposed so as to enter the conveying path from the first main surface 10s side of the main sheet 10 being conveyed along the conveying path, at the first position 3a on the conveying path. Thereby, as shown in Figs. 2B and 3, in the non-woven fabric sheet 14 of the main sheet 10 being conveyed, slits 16 are formed so that incisions 17 are formed in the first main surface 10s of the main sheet 10. The incisions 17 and the slits 16 are formed so as to be continuous in the longitudinal direction of the main sheet 10.

More specifically, the single-edged blades 60k of the tools 60 are disposed so as to enter the conveying path only up to in front of the second main surface 10t of the main sheet 10 being conveyed along the conveying path, from the first main surface 10s side of the main sheet 10 being conveyed along the conveying path, at the first position 3a on the conveying path. Thereby, the single-edged blades 60k of the tools 60 form the slits 16 only from the first main surface 10s up to in front of the second main surface 10t of the main sheet 10 being conveyed along the conveying path.

The slit forming device may use a round blade as in a second modification described later, or may be a device that forms slits by a method such as laser or water jet. Further, it may be a device that manufactures the main sheet 10 where slits are formed in the non-woven fabric sheet 14, by arranging a plurality of belt-like non-woven fabric sheets so as to adjoin each other in the width direction and forming them into one sheet.

The dispersing device 7 disperses, as shown in Fig. 2C, the particulate matter 11 from the main sheet 10s side to the main sheet 10 where the slits 16 are formed. The particulate matter 11 is powdery and/or granular one capable of absorbing and retaining liquid. The particulate matter 11 contains, for example, a superabsorbent polymer (SAP) as a major ingredient. As the particulate matter 11, for example, SAP particles whose average particle diameter is approximately 40 µm to approximately 850 µm are preferable. Fine particles with a diameter smaller than this may be contained.

As shown in Fig. 1, the dispersing device 7 includes: a drum 70 having a cylindrical support surface 70s that supports the second main surface 10t of the main sheet 10; a particulate matter supply device 72 that is opposed to the first main surface 10s of the part, of the main sheet 10, abutting on the support surface 70s so as to be supported thereby, and disperses the particulate matter 11 with predetermined timing; a plurality of tanks 76a and 76b storing the particulate matter 11; and an amount adjusting device 74 that adjusts the amount and mixing ratio of the particulate matter 11 supplied from the tanks 76a and 76b to the particulate matter supply device 72.

Fig. 4 is a plan view of the main sheet 10. The particulate matter supply device 72 disperses the particulate matter 11 only into dispersion regions 15 surrounded by the chain lines in Fig. 4. No particulate matter 11 is dispersed into gaps 15x between the adjoining dispersion regions 15. For this reason, by cutting at these gaps 15x, individual pieces of the absorbent body can be taken out without any influence of the particulate matter 11.

As shown in Fig. 1, the covering device 8 includes a first and second guide rolls 8a and 8b. The first guide roll 8a guides the covering sheet 18. The second guide roll 8b is disposed so as to adjoin the drum 70, overlays the covering sheet 18 on the first main surface 10s of the part, of the main sheet 10, supported by the support surface 70s of the drum 70, and presses the covering sheet 18 against the first main surface 10s of the main sheet 10. Since the covering sheet 18 is conveyed together with the main sheet 10 when overlaid on the first main surface 10s of the main sheet 10, the part of the covering sheet 18 before overlaid on the main sheet 10 is conveyed in the direction indicated by the arrow 18x.

The covering sheet 18 is a sheet that prevents passage of the particulate matter 11. The covering sheet 18 may allow passage of liquid or may be a sheet that prevents passage of liquid. For the covering sheet 18, for example, tissue paper whose thickness is small compared with the main sheet 10 is used. For the covering sheet 18, there are also cases where non-woven fabric is used and where an opaque back sheet formed of a film is pasted to one surface.

As shown in Fig. 2D, the width of the covering sheet 18 is larger than that of the main sheet 10, and the covering sheet 18 has protruding portions 18a and 18b that protrude on both sides of the main sheet 10 in the width direction when the covering sheet 18 is overlaid on the main sheet 10.

As shown in Fig. 1, the absorbent body manufacturing device 2 is further provided with a first and second adhesive agent applying devices 9a and 9b and a bending device 9k. The first adhesive agent applying device 9a applies an adhesive agent (for example, hot melt) in a predetermined pattern to one of a pair of main surfaces of the covering sheet 18 that is pasted to the main sheet 10. The second adhesive agent applying device 9b applies an adhesive agent (for example, hot melt) in a predetermined pattern to the protruding portions 18a and 18b of the covering sheet 18 (see Fig. 2D). As shown in Fig. 2E, the bending device 9k bends the protruding portions 18a and 18b of the covering sheet 18 and overlays them on the second main surface 10t.

The main sheet 10 and the covering sheet 18 pass between the drum 70 and the second guide roll 8b and further pass between the pair of press contact rolls 4p and 4q, whereby the non-woven fabric sheet 14 is gradually compressed to be thinned.

Although not shown, an adhesive agent such as hot melt may be applied to the first main surface 10s of the main sheet 10 before the particulate matter 11 is dispersed. In this case, it is preferable to apply the adhesive agent after slits are formed in the non-woven fabric sheet 14 of the main sheet 10 because the adhesive agent does not stick to the single-edged blades 60k of the tools 60.

Next, a method for manufacturing the absorbent body 20 by using the absorbent body manufacturing device 2 of the first embodiment will be described with reference to Figs. 1 and 2A to 2E.

First, by the conveying device 4, the main sheet 10 is conveyed in the longitudinal direction of the main sheet 10 along a predetermined conveying path. This is a first step.

Then, by the slit forming device 6, the slits 16 are formed so that the incisions 17 are formed in the first main surface 10s as shown in Fig. 2B at the first position 3a on the conveying path. This is a second step.

Then, by the dispersing device 7, the particulate matter 11 capable of absorbing and retaining liquid is dispersed into a region, including the incisions 17, of the first main surface 10s of the main sheet 10 being conveyed as shown in Fig. 2C at the second position 3b on the conveying path. This is a third step.

Then, by the covering device 8, the covering sheet 18 that prevents passage of the particulate matter 11 is overlaid on the region, where the particulate matter 11 has been dispersed, of the first main surface 10s of the main sheet 10 being conveyed as shown in Fig. 2D at the third position 3c on the conveying path. This is a fourth step.

By the above-described first to fourth steps, the basic structure of the absorbent body 20 is completed.

Then, by the bending device 9k, the protruding portions 18a and 18b of the covering sheet 18 protruding on both sides of the main sheet 10 in the width direction are bent, and the protruding portions 18a and 18b are overlaid on the second main surface 10t of the main sheet 10 as shown in Fig. 2E. Then, the absorbent body 20 is thinned by the pair of press contact rolls 4p and 4q.

Then, the absorbent body 20 as a continuous body is cut with a predetermined pitch in the conveying direction to take out individual pieces of the absorbent body 20.

By the above-described steps, pieces of the absorbent body 20 can be manufactured.

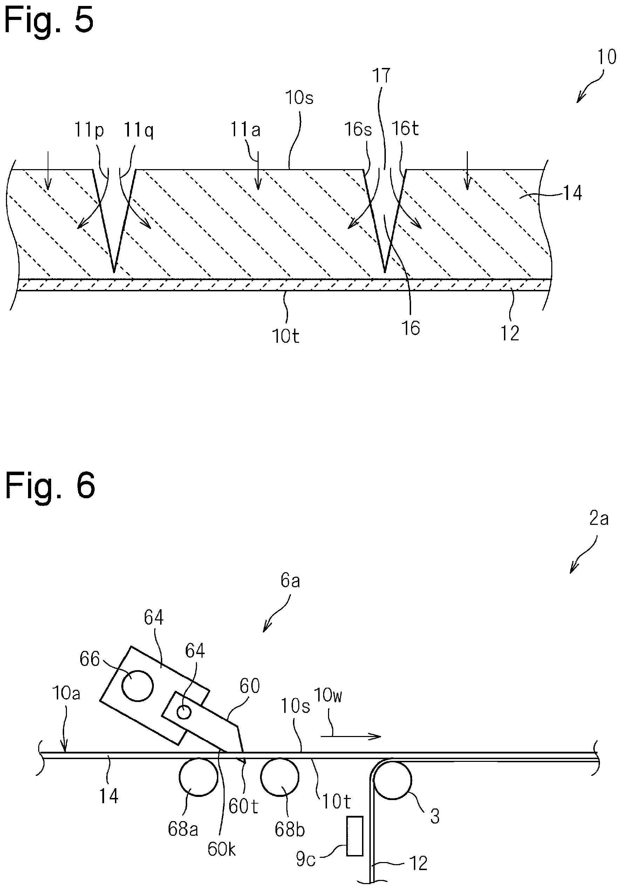

Fig. 5 is a cross-sectional view of the main sheet 10 which is cut in the width direction of the main sheet 10. As shown in Fig. 5, at the second step of forming the slits 16, the slits 16 are formed only in the non-woven fabric sheet 14 and are not formed in the support sheet 12.

At the third step of dispersing the particulate matter, the particulate matter dispersed to the first main surface 10s side of the main sheet 10 moves to the inside of the non-woven fabric sheet 14. At this time, part of the dispersed particulate matter enters the inside of the non-woven fabric sheet 14 from the first main surface 10s as indicated by the arrow 11a, and the remaining part enters the slits 16 from the incisions 17 as indicated by the arrows 11p and 11q to enter the inside of the non-woven fabric sheet 14 from cut surfaces 16s and 16t of the slits 16.

That is, the dispersed particulate matter enters the non-woven fabric sheet 14 also from the cut surfaces 16s and 16t of the slits 16 as well as the first main surface 10s and becomes tangled with the fibers of the non-woven fabric sheet 14 to be held in the voids between the fibers. For this reason, the amount of particulate matter held in the non-woven fabric sheet 14 can be easily increased compared with when no slits 16 are formed.

In a case where no slits 16 are formed in the non-woven fabric sheet 14, since the fibers are complicatedly tangled in the non-woven fabric sheet 14, when the liquid supplied to the surface of the absorbent body 20 reaches the non-woven fabric sheet 14, the liquid is absorbed and retained by the particulate matter held in the non-woven fabric sheet 14 while substantially uniformly spreading gradually in the surface direction of the non-woven fabric sheet 14.

On the other hand, in a case where the slits 16 are formed, the fibers complicatedly tangled in the non-woven fabric sheet 14 are cut at the parts where the slits 16 are formed. For this reason, when the liquid supplied to the surface of the absorbent body 20 reaches the non-woven fabric sheet 14, the liquid quickly moves and spreads along the slits 16 formed in the non-woven fabric sheet 14 and at the same time, part of the liquid that moves along the slits 16 is diffused in the non-woven fabric sheet 14 in a direction away from the slits 16. The diffused liquid is absorbed and retained by the particulate matter held by the non-woven fabric sheet 14.

For this reason, with the absorbent body 20 where the slits 16 are formed in the non-woven fabric sheet 14, liquid of the same amount can be easily made to be absorbed and retained by the particulate matter in a wider range in a shorter time compared with when no slits 16 are formed.

Consequently, the absorbent body 20 with which it is easy to enhance liquid permeability and liquid diffusibility can be manufactured.

While Fig. 5 schematically illustrates a case where the cut surfaces 16s and 16t of the slits 16 are away from each other, the cut surfaces 16s and 16t may be such that part or the whole thereof are in contact with each other.

The gaps between the slits 16 (the spaces between the cut surfaces 16s and 16t of the slits 16) may be widened by tension acting on the non-woven fabric sheet 14 where the plurality of slits 16 are formed in the conveying direction (longitudinal direction) so that the parts between the adjoining slits 16 of the non-woven fabric sheet 14 are thinned. If the gaps of the slits 16 are wide, not only the passage and diffusion of the liquid are excellent but also the particulate matter easily enters the inside of the non-woven fabric sheet 14 from the slits 16.

While the slits 16 do not penetrate the non-woven fabric sheet 14 in Fig. 5, for example, slits that penetrate the non-woven fabric sheet 14 may be formed as in a first modification described later.

The slits 16 extending in the longitudinal direction of the main sheet 10 as in the first embodiment can be easily formed by a simple structure or method such as squeezing the single-edged blades 60k of the tools 60 into the main sheet 10 being conveyed.

However, the slits 16 may be formed in an arbitrary direction. For example, by using a cylindrical cutting roll where linear or spiral blades are formed, slits orthogonal to the longitudinal direction of the main sheet 10 or slits intersecting the longitudinal direction can be formed.

While the slits 16 of the first embodiment do not penetrate the main sheet 10, slits can be formed that penetrate between the first main surface 10s and the second main surface 10t of the main sheet 10. However, when slits penetrating the main sheet 10 are formed, there are cases where the dispersed particulate matter passes through the slits, so that the particulate matter having passed through the slits are not held in the non-woven fabric sheet 14.

On the contrary, when the slits 16 are formed only from the first main surface 10s up to in front of the second main surface 10t of the main sheet 10 as in the first embodiment, it is possible to make all the dispersed particulate matter 11 held in the non-woven fabric sheet 14, so that the amount of particulate matter 11 held in the non-woven fabric sheet 14 can be increased. Moreover, since the conveyance of the main sheet 10 is easy, it is easy to increase the manufacturing capacity of the absorbent body 20 by increasing the conveying speed of the main sheet 10.

The shape of the slits 16 is not limited to a straight line. For example, by swinging the blades in the width direction of the main sheet, curved slits can be formed.

Next, a first to twelfth modifications of the first embodiment will be described. In the following, the same components are denoted by the same reference numerals and differences from the first embodiment are mainly described.

### <First modification>

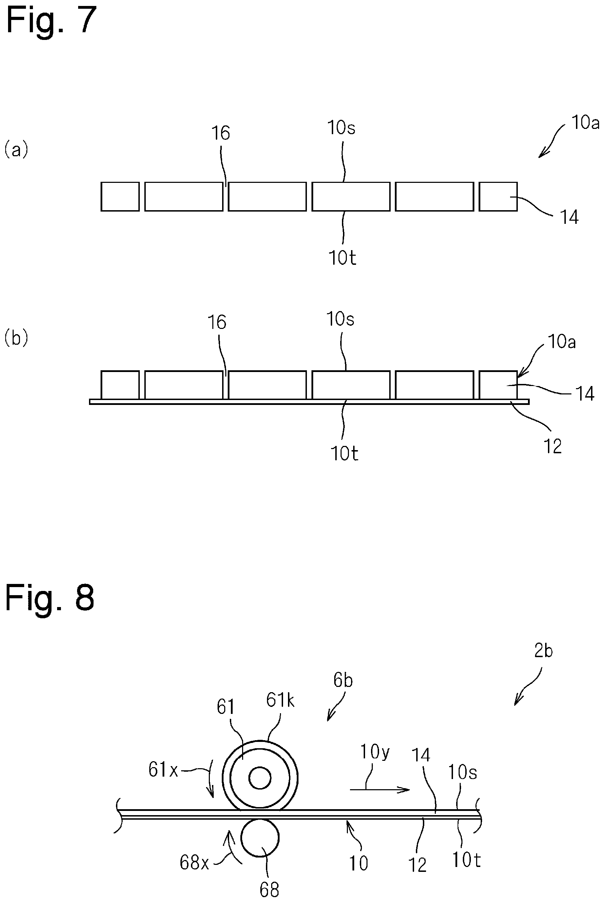

A modification where slits penetrating a main sheet 10a are formed will be described with reference to Figs. 6, 7A and 7B.

Fig. 6 is a schematic view showing a relevant part structure of an absorbent body manufacturing device 2a of the first modification. Figs. 7A and 7B are cross-sectional views of the main sheet 10a which is cut in the width direction. As shown in Figs. 6 and 7A, the main sheet 10a is constituted by only the non-woven fabric sheet 14, and the first main surface 10s and the second main surface 10t of the main sheet 10a are both constituted by the non-woven fabric sheet 14. The main sheet 10a passes a slit forming device 6a having a substantially similar structure to that of the first embodiment and where the tools 60 are disposed.

As shown in Fig. 6, unlike in the first embodiment, the tools 60 are disposed so that the ends 60t of the single-edged blades 60k of the tools 60 protrude toward the opposite side of the conveying path of the main sheet 10a, and the single-edged blades 60k of the tools 60 cross the conveying path of the main sheet 10a. For this reason, as shown in Fig. 7A, the slits 16 that penetrate the non-woven fabric sheet 14 are formed in the non-woven fabric sheet 14 of the main sheet 10a conveyed in the direction indicated by the arrow 10w.

On the side where the ends 60t of the single-edged blades 60k of the tools 60 protrude, a pair of receiving rolls 68a and 68b are disposed adjacently to the conveying path of the main sheet 10a so as to be in contact with the second main surface 10t of the main sheet 10a. The ends 60t of the tools 60 protrude between the pair of receiving rolls 68a and 68b.

While it is preferable to provide the receiving rolls 68a and 68b because the non-woven fabric sheet 14 can be firmly supported and the slits 16 are easily formed in the non-woven fabric sheet 14, a structure having no receiving roll 68a or 68b may be adopted.

On the downstream side of the slit forming device 6a in the conveying direction of the main sheet 10a, an introducing roll 3 is disposed that overlays the support sheet 12 on the second main surface 10t of the main sheet 10a constituted by only the non-woven fabric sheet 14. Moreover, on the upstream side of the introducing roll 3 in the conveying direction of the support sheet 12, an adhesive agent applying device 9c is provided that applies an adhesive agent (for example, hot melt) to the surface of the support sheet 12 to be pasted to the non-woven fabric sheet 14.

After the non-woven fabric sheet 14 and the support sheet 12 are pasted together as shown in Fig. 7B, an absorbent body is manufactured by similar steps to those of the first embodiment.

### <Second modification>

A second modification in which a slit is formed by using a tool 61 having a round blade 61k will be described with reference to Fig. 8.

Fig. 8 is a schematic view showing a relevant part structure of an absorbent body manufacturing device 2b of the second modification. As shown in Fig. 8, in a slit forming device 6b of the absorbent body manufacturing device 2b, the tool 61 having the round blade 61k as a circularly continuous blade is disposed on the first main surface 10s side of the main sheet 10 conveyed in the direction indicated by the arrow 10y, and the receiving roll 68 opposed to the tool 61 is disposed on the second main surface 10t side of the main sheet 10. The tool 61 and the receiving roll 68 may be structured so as to be rotated in the directions indicated by the arrows 61x and 68x or to be idled.

The round blade 61k of the tool 61 is disposed so that a predetermined gap is provided from the receiving roll 68. By the main sheet 10 passing between the tool 61 and the receiving roll 68, a continuous slit is formed in the non-woven fabric sheet 14.

While it is preferable to provide the receiving roll 68 because the main sheet 10 can be firmly supported and the slit is easily formed in the non-woven fabric sheet 14 of the main sheet 10, a structure having no receiving roll 68 may be adopted.

### <Third modification>

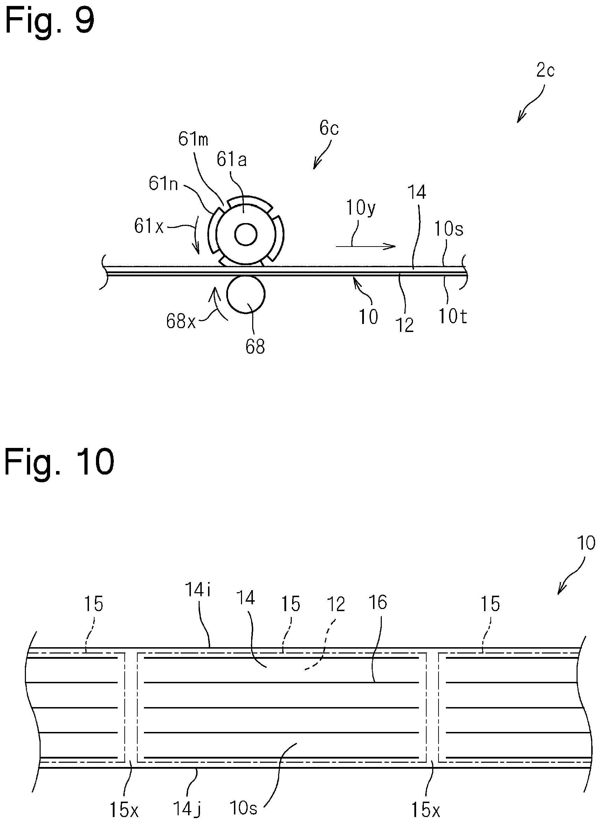

A third modification in which slits are discontinuously or intermittently formed will be described with reference to Fig. 9.

Fig. 9 is a schematic view showing a relevant part structure of an absorbent body manufacturing device 2c of a third modification. While as shown in Fig. 9, a slit forming device 6c of the absorbent body manufacturing device 2c is structured substantially similarly to the slit forming device 6b of the second modification, unlike in the second modification, a tool 61a has a plurality of arc-shaped blades 61n disposed at intervals 61m in the circumferential direction.

By the main sheet 10 passing between the tool 61a and the receiving roll 68, a plurality of slits adjoining at intervals therebetween in the conveying direction (longitudinal direction) of the main sheet 10 are formed in the non-woven fabric sheet 14 of the main sheet 10.

Discontinuous slits can also be formed, for example, with a structure in which a blade such as a single-edged blade or a round blade repeats entering and retreating from the conveying path of the main sheet.

### <Fourth modification>

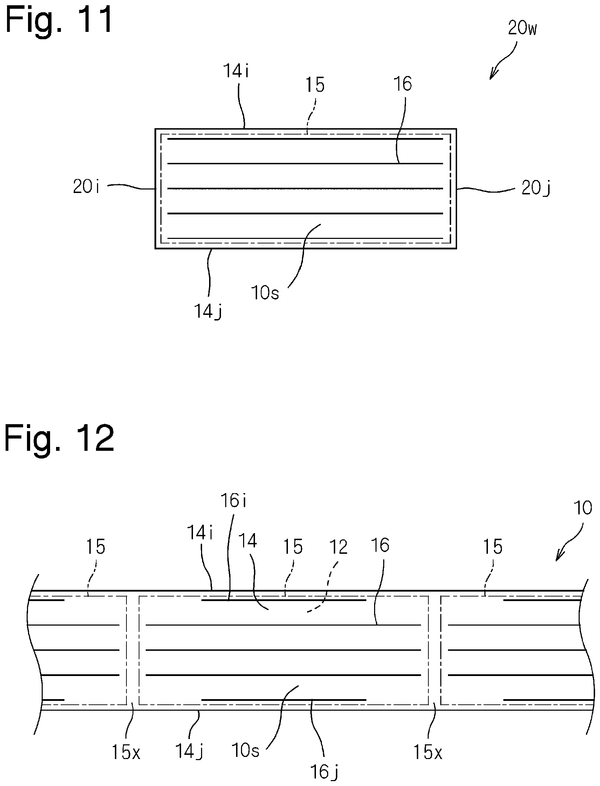

A fourth modification in which slits are discontinuously or intermittently formed so that the incisions 17 are formed only in dispersion regions will be described with reference to Figs. 10 and 11.

Fig. 10 is a perspective view of the main sheet 10 perspectively viewed vertically to the first main surface 10s. In Fig. 10, the chain lines indicate the dispersion regions 15 where the particulate matter is to be dispersed. As shown in Fig. 10, the slits 16 are formed only in the dispersion regions 15 separated from width direction side edges 14i and 14j of the non-woven fabric sheet 14 of the main sheet 10 and are not formed in the gaps 15x between the adjoining dispersion regions 15.

By cutting the absorbent body as a continuous body manufactured by using this main sheet 10, an absorbent body piece 20w shown in Fig. 11 can be obtained.

Fig. 11 is a perspective view of the absorbent body piece 20w perspectively viewed vertically to the first main surface 10s of the main sheet 10. As shown in Fig 11, the slits 16 do not reach end portions 20i and 20j of the absorbent body piece 20w.

By forming the slits 16 only within the dispersion regions 15, in the post-process during manufacture, the possibility can be reduced that the particulate matter having entered the slits 16 moves along the slits 16 and spills out from the end portions 20i and 20j of the absorbent body piece 20w. When liquid moves along the slits 16 in the absorbent body piece 20w, since the liquid does not move up to the region outside the dispersion region 15, that is, the region where no particulate matter is dispersed, the liquid having moved along the slits 16 is surely absorbed by the particulate matter.

### <Fifth modification>

A fifth modification in which a plurality of slits formed discontinuously or intermittently are formed so as to have different lengths will be described with reference to Fig. 12.

Fig. 12 is a perspective view of the main sheet 10 seen through vertically to the first main surface 10s like Fig. 10. In Fig. 12, the chain lines indicate the dispersion regions 15 where the particulate matter is to be dispersed. As shown in Fig. 12, the slits 16 in and around the center of the main sheet 10 in the width direction of the non-woven fabric sheet 14 are made longer than slits 16i and 16j close to the width direction side edges 14i and 14j of the non-woven fabric sheet 14 of the main sheet 10. Since the liquid is discharged to the neighborhood of the center, the liquid can be made to easily spread by the slits 16.

### <Sixth modification>

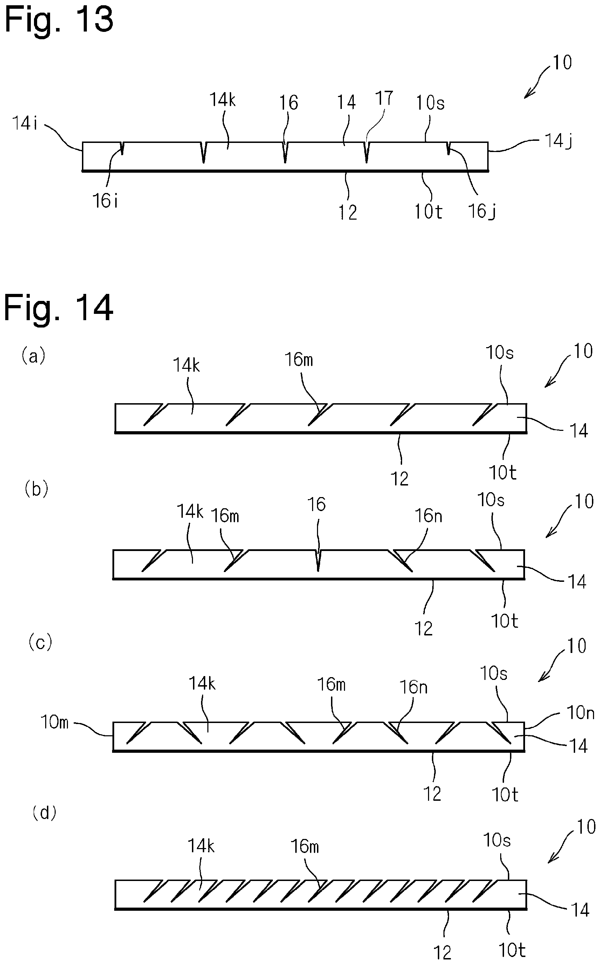

A sixth modification in which slits having different depths are formed will be described with reference to Fig. 13.

Fig. 13 is a cross-sectional view of the main sheet 10 which is cut in the width direction. As shown in Fig. 13, a plurality of slits 16, 16i and 16j are formed in the bulky structure 14k of the non-woven fabric sheet 14 of the main sheet 10, and the central slit 16 is formed so as to be deeper than the slits 16i and 16j close to the width direction side edges 14i and 14j. Since liquid is discharged to the center, the liquid can be made to easily spread by the deeper slit 16.

### <Seventh modification>

A seventh modification in which slits are formed in a direction inclined with respect to the first main surface 10s of the main sheet 10 will be described with reference to Figs. 14A to 14D. Figs. 14A to 14D are cross-sectional views of the main sheet 10 which is cut in the width direction.

In Fig. 14A, slits 16m inclined with respect to the first main surface 10s are formed in the bulky structure 14k of the non-woven fabric sheet 14 of the main sheet 10.

In Fig. 14B, a slit 16 is formed in a direction orthogonal to the first main surface 10s in the center in the width direction of the main sheet 10, and on both sides thereof, slits 16m and 16n are formed that are inclined in such a way that they are away from the central slit 16 as they approach the second main surface 10t.

In Fig. 14C, in the width direction of the main sheet 10, first slits 16m that approach one width direction side edge 10m as they approach the second main surface 10t and second slits 16n that approach the other width direction side edge 10n as they approach the second main surface 10t are formed alternately in the width direction.

In Fig. 14D, a multiplicity of slits 16m in an inclined direction similar to those of the Fig. 14A are formed. When perspectively viewed vertically to the first main surface 10s of the main sheet 10, the slits 16m occupy most part (for example, an area of not less than 50%) of the first main surface 10s of the main sheet 10.

In the case of the slits 16m and 16n formed in a direction inclined with respect to the first main surface 10s of the main sheet 10, the slit length in the thickness direction of the sheet can be made large and the particulate matter easily enters the non-woven fabric, so that the diffusion of liquid is easy. Further, since the slits 16m and 16n spread in the width direction, the particulate matter and the liquid easily spread in the width direction along the slits 16m and 16n, so that the entire area of the absorbent body can be effectively utilized.

### <Eighth modification>

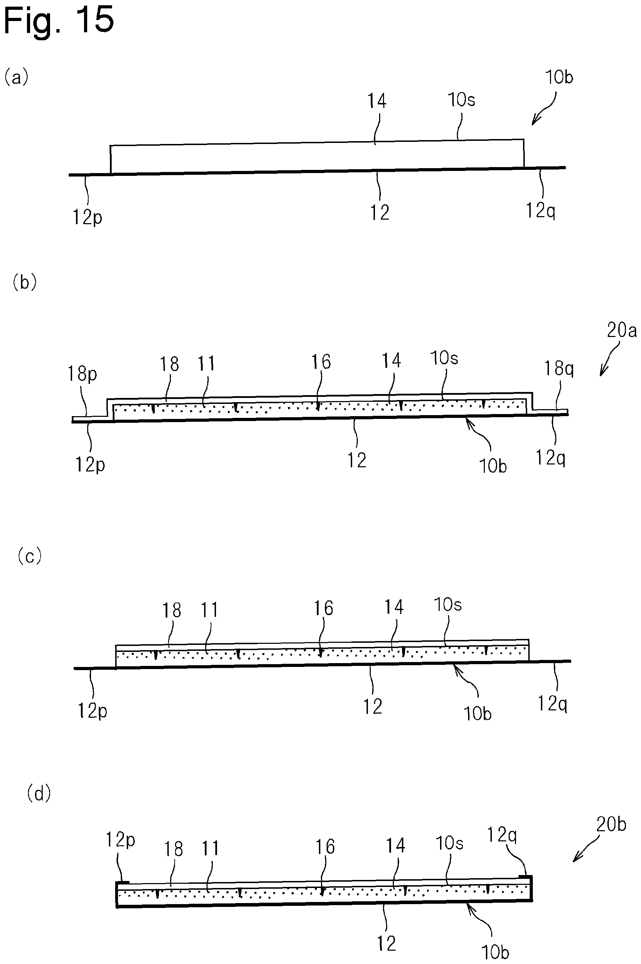

An eighth modification using a main sheet 10b where the support sheet 12 protrudes on both sides in the width direction of the non-woven fabric sheet 14 will be described with reference to Figs. 15A to 15D.

Figs. 15A to 15D are cross-sectional views of the main sheet 10b and absorbent bodies 20a and 20b which are cut in the width direction. As shown in Fig. 15A, in the main sheet 10b, the width of the support sheet 12 is larger than that of the non-woven fabric sheet 14, and the support sheet 12 has protruding portions 12p and 12q protruding on both sides of the non-woven fabric sheet 14. The main surface 10s of the main sheet 10b is constituted by the non-woven fabric sheet 14 and the protruding portions 12p and 12q of the support sheet 12.

As shown in Fig. 15B, the covering sheet 18 having the same width as the support sheet 12 is overlaid on the non-woven fabric sheet 14 where the slits 16 have been formed and the particulate matter 11 has been dispersed and the protruding portions 12p and 12q of the support sheet 12, and these are pasted together. Thereby, the absorbent body 20a can be manufactured.

Alternatively, as shown in Fig. 15C, the covering sheet 18 having the same width as the non-woven fabric sheet 14 is overlaid on the non-woven fabric sheet 14 where the slits 16 have been formed and the particulate matter 11 has been dispersed. Then, as shown in Fig. 15D, the protruding portions 12p and 12q of the support sheet 12 are bent and overlaid on the covering sheet 18, and these are pasted together. Thereby, the absorbent body 20b can be manufactured.

### <Ninth modification>

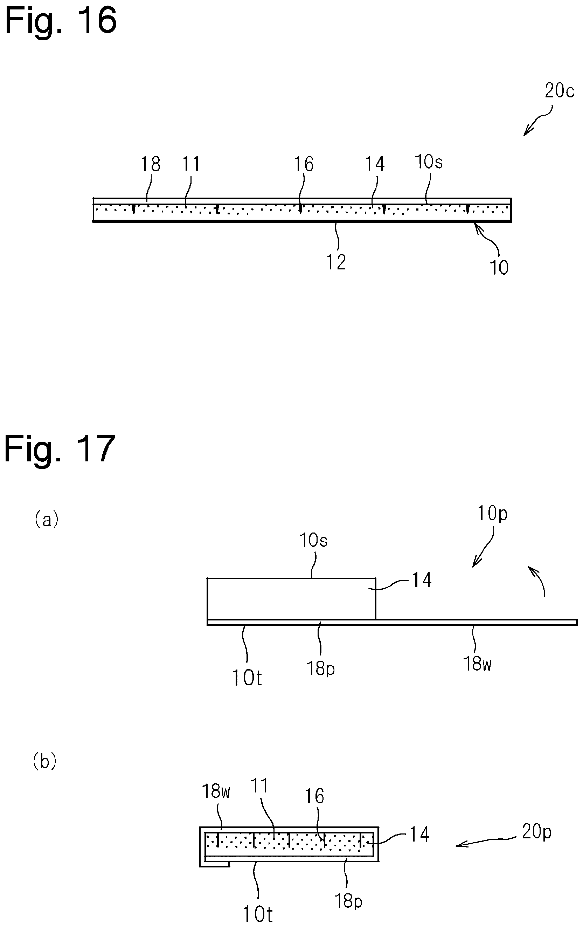

A ninth modification in which an absorbent body 20c is manufactured without the support sheet 12 or the covering sheet 18 being bent will be described with reference to Fig. 16.

Fig. 16 is a cross-sectional view of the absorbent body 20c which is cut in the width direction. As shown in Fig. 16, the support sheet 12 and the non-woven fabric sheet 14 of the main sheet 10 have the same width. The covering sheet 18 having the same width as the main sheet 10 is overlaid on the non-woven fabric sheet 14 where the slits 16 have been formed and the particulate matter 11 has been dispersed, and these are pasted together. Thereby, the absorbent body 20c can be manufactured.

### <Tenth modification>

Three tenth modifications in which absorbent bodies 20p, 20q and 20r are manufactured by using main sheets 10p, 10q and 10r including covering sheets 18p, 18q and 18r will be described with reference to Figs. 17A to 19B.

Figs. 17A and 17B are cross-sectional views of the main sheet 10p and the absorbent body 20p which are cut in the width direction. As shown in Fig. 17A, in the main sheet 10p, the covering sheet 18p is pasted to the non-woven fabric sheet 14. The width of the covering sheet 18p is larger than that of the non-woven fabric sheet 14, and the covering sheet 18p has a protruding portion 18w protruding on one side of the non-woven fabric sheet 14 in the width direction. The first main surface 10s of the main sheet 10p is constituted by the non-woven fabric sheet 14, and the second main surface 10t of the main sheet 10p is constituted by the covering sheet 18p.

As shown in Fig. 17B, the protruding portion 18w of the covering sheet 18p is bent in the direction indicated by the arrow shown in Fig. 17A and overlaid on the non-woven fabric sheet 14 where the slits 16 have been formed and the particulate matter 11 has been dispersed and further, is bent in such a way that the end side of the protruding portion 18w of the covering sheet 18p is overlaid on the second main surface 10t of the main sheet 10, and these are pasted together. Thereby, the absorbent body 20p can be manufactured.

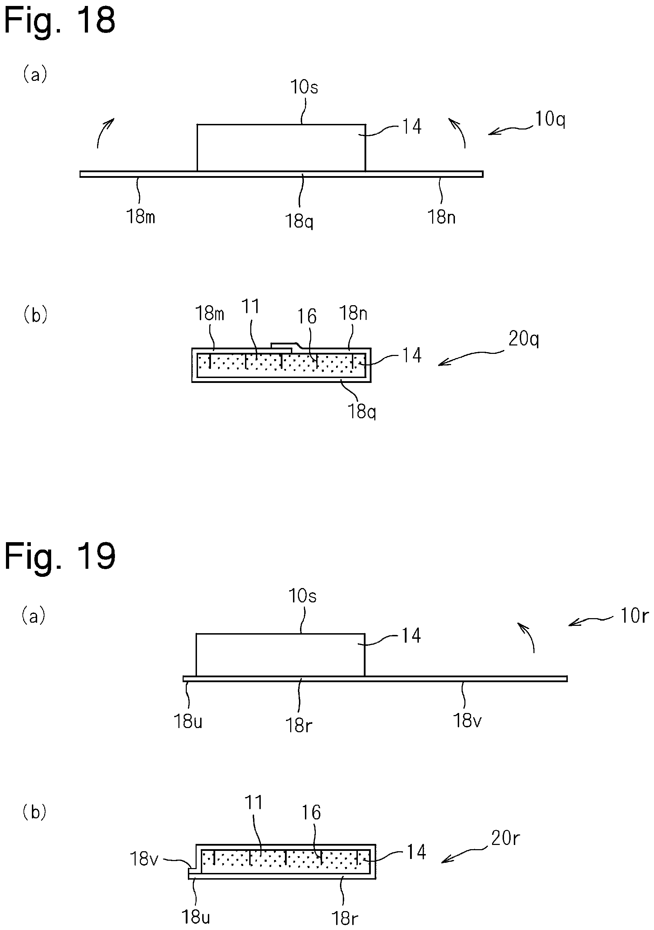

Figs. 18A and 18B are cross-sectional views of the main sheet 10q and the absorbent body 20q which are cut in the width direction. As shown in Fig. 18A, in the main sheet 10q, the width of the covering sheet 18q is larger than that of the non-woven fabric sheet 14, and the covering sheet 18q has protruding portions 18m and 18n substantially equally protruding on both sides of the non-woven fabric sheet 14.

As shown in Fig. 18B, the protruding portions 18m and 18n of the covering sheet 18q are bent in the directions of the arrows shown in Fig. 18A and overlaid on the non-woven fabric sheet 14 where the slits 16 have been formed and the particulate matter 11 has been dispersed, and these are pasted together. Thereby, the absorbent body 20q can be manufactured.

Figs. 19A and 19B are cross-sectional views of the main sheet 10r and the absorbent body 20r which are cut in the width direction. As shown in Fig. 19A, in the main sheet 10r, the width of a covering sheet 18r is larger than that of the non-woven fabric sheet 14, and the covering sheet 18r has a protruding portion 18u slightly protruding on one side of the non-woven fabric sheet 14 and a protruding portion 18v largely protruding on the other side of the non-woven fabric sheet 14.

As shown in Fig. 19B, on the non-woven fabric sheet 14 where the slits 16 have been formed and the particulate matter 11 has been dispersed and on one protruding portion 18u of the covering sheet 18r, the other protruding portion 18v of the covering sheet 18r is bent in the direction shown by the arrow shown in Fig. 19A and overlaid, and these are pasted together. Thereby, the absorbent body 20r can be manufactured.

### <Eleventh modification>

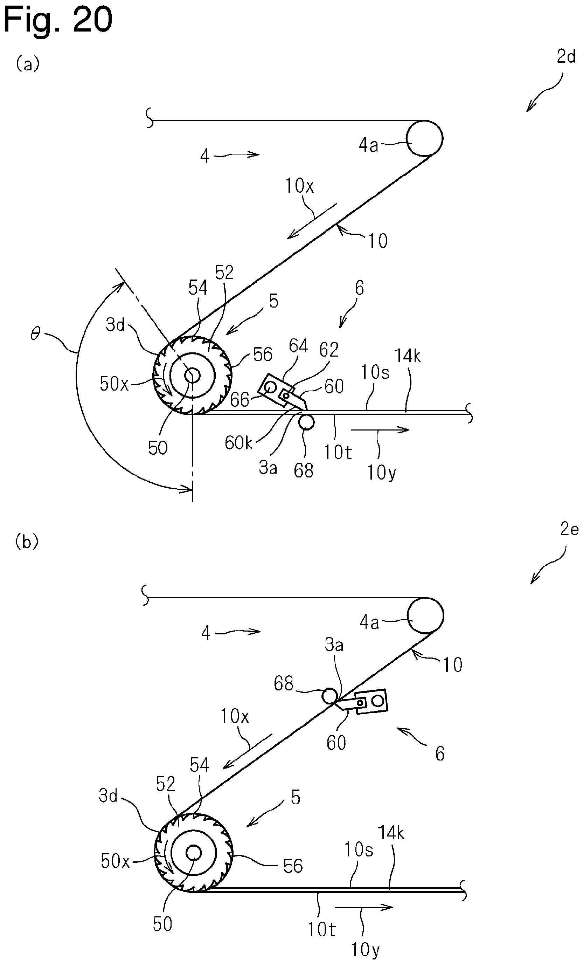

An eleventh modification in which the bulky structure 14k is formed on the non-woven fabric sheet of the main sheet 10 during the conveyance of the main sheet 10 will be described with reference to Figs. 20A and 20B.

Fig. 20A is a schematic view showing a relevant part structure of an absorbent body manufacturing device 2d. As shown in Fig 20A, in the absorbent body manufacturing device 2d, a fiber raising device 5 is disposed so as to adjoin a fourth position 3d on the conveying path of the main sheet 10 conveyed in the direction shown by the arrows 10x and 10y by the conveying device 4. The fourth position 3d is on the upstream side, in the conveying direction of the main sheet 10, of the first position 3a that the slit forming device 6 is disposed so as to adjoin.

The fiber raising device 5 raises the fibers of the non-woven fabric sheet of the main sheet 10 along the first main surface 10s of the main sheet 10 to form the bulky structure 14k on the non-woven fabric sheet constituting at least part of the first main surface 10s of the main sheet 10.

Specifically, the fiber raising device 5 is provided with a tiller 56 in which a plurality of rotary blades 52 having a plurality of blades 54 in the circumferential direction are fixed to a rotation shaft 50 so that the positions of the blades 54 of the adjoining rotary blades 52 are shifted from each other. The fiber raising device 5 is disposed so that the cylindrical peripheral surface of the tiller 56 formed of the tips of the blades 54 of the rotary blades 52 abuts on the first main surface 10s of the main sheet 10.

The rotation shaft 50 is rotated, for example, in the direction shown by the arrow 50x in such a way that there is a difference between the movement speed of the tips of the blades 54 of the rotary blades 52 (hereinafter, referred to as "peripheral speed of the tiller 56") and the movement speed of the first main surface 10s of the main sheet 10 (hereinafter, referred to as "conveying speed of the main sheet 10"). Thereby, the blades 54 of the rotary blades 52 moving at a different speed from the main sheet 10 in the same direction as the main sheet 10 along the main sheet 10 scratches the non-woven fabric sheet constituting the first main surface 10s of the main sheet 10 and raises the fibers of the non-woven fabric sheet to make the first main surface 10s side of the main sheet 10 bulky. That is, the fibers of the non-woven fabric sheet are raised along the first main surface 10s, whereby the bulky structure 14k is formed.

That is, the fiber raising device 5 forms the bulky structure 14k constituting at least part of the first main surface 10s of the main sheet 10 and having voids allowing the particulate matter to enter, on the non-woven fabric sheet of the main sheet 10 being conveyed by the conveying device 4 at the fourth position 3d on the conveying path on the upstream side, in the conveying direction of the main sheet 10, of the second position 3b (see Fig. 1) on the conveying path. This is a fifth step.

Since the amount of particulate matter 11 held in the non-woven fabric sheet can be increased by forming the fiber-raised bulky structure 14k suitable for holding the particulate matter 11 at the fifth step using the fiber raising device 5, it is easy to enhance liquid permeability and liquid diffusibility.

The manner in which the tiller 56 and the non-woven fabric sheet constituting the first main surface 10s of the main sheet 10 are in contact with each other is not limited to the mode of Fig. 20A but may be arbitrarily selected. For example, when a receiving roll is provided on the side opposite to the tiller 56 with the main sheet 10 in between, a structure may be adopted in which the main sheet 10 is conveyed from the tangential direction with respect to the peripheral surface of the tiller 56 and made to pass between the tiller 56 and the receiving roll.

On the other hand, when the main sheet 10 is wound on part of the peripheral surface of the tiller 56 as shown in Fig. 20A, since the main sheet 10 is pressed against the tiller 56 by the tension at the time of conveyance, the above-mentioned receiving roll can be omitted. In this case, the embracing angle θ of the tiller 56, that is, the central angle θ of the tiller 56 in the section where the main sheet 10 is in contact with the tiller 56 is set, for example, within a range of approximately 30 to 180 degrees according to fiber raising conditions such as the material and basis weight of the main sheet 10 and the peripheral speed of the tiller 56.

Regarding the peripheral speed of the tiller 56 with respect to the conveying speed of the main sheet 10, normally, the larger the difference is, the more easily the non-woven fabric sheet of the main sheet 10 is fiber-raised to become bulky; however, if the difference is excessively large, the load on the main sheet 10 is high, so that there is a fear that the main sheet 10 becomes deformed or is cut. For this reason, the ratio of the peripheral speed of the tiller 56 to the conveying speed of the main sheet 10 is set, for example, within a range of 50 to 90% so that the non-woven fabric sheet of the main sheet 10 has an appropriate bulkiness according to the material and basis weight of the main sheet 10 used, the shape, structure and embracing angle θ of the blades of the tiller 56, and the like.

For example, when a main sheet 10 with a basis weight of 150 g/m² and a thickness of approximately 4 mm is used, if the above-mentioned embracing angle θ is set to 50 to 70 degrees and the peripheral speed of the tiller 56 is set to 70 to 90% of the conveying speed of the main sheet 10, the thickness of the main sheet 10 becomes approximately 1.5 to 2.0 times as bulky as the original thickness, and if the peripheral speed of the tiller 56 is set to 50 to 60% of the conveying speed of the main sheet 10, the thickness of the main sheet 10 becomes 2.5 to 3.5 times as bulky as the original thickness.

When the particulate matter (SAP particles) is dispersed at the above-described third step on the main sheet 10 having become bulky as described above, the bulkier the main sheet 10 is, the higher the ratio of the particulate matter, of the dispersed particulate matter, that enters the central part and the lower part of the main sheet 10 in the thickness direction.

Even if the peripheral speed of the tiller 56 is higher than the conveying speed of the main sheet 10, since there is a difference in speed between the main sheet 10 and the tiller 56, the non-woven fabric sheet of the main sheet 10 can be fiber-raised so as to be bulky. In this case, the orientation of the hook-like parts of the blades 54 of the rotary blades 52 is made opposite to that when the peripheral speed of the tiller 56 is lower than the conveying speed of the main sheet 10. The peripheral speed of the tiller 56 in this case is set, for example, within a range of not more than 130% of the conveying speed of the main sheet 10 so that the non-woven fabric sheet of the main sheet 10 has an appropriate bulkiness according to the material and basis weight of the main sheet 10 used, the shape, structure and embracing angle θ of the tiller 56, and the like.

Fig. 20B is a schematic view showing a relevant part structure of an absorbent body manufacturing device 2e having another structure. As shown in Fig. 20B, in the absorbent body manufacturing device 2e, the fourth position 3d that the fiber raising device 5 is disposed so as to adjoin is on the downstream side, in the conveying direction of the main sheet 10, of the first position 3a that the slit forming device 6 is disposed so as to adjoin. In this case, after slits are formed, the bulky structure 14k is formed.

As still another structure, for example, a structure may be adopted in which the round blade 61k as in the second modification is attached to the fiber raising device 5 together with the rotary blades 52 and the formation of the bulky structure 14k and the formation of slits are performed at the same time. In this case, the fourth position 3d at which the bulky structure is formed and the first position 3a at which slits are formed are the same position.

In any structure, the fiber raising device 5 is disposed so as to adjoin the fourth position 3d on the upstream side, in the conveying direction of the main sheet 10, of the second position 3b.

### <Twelfth modification>

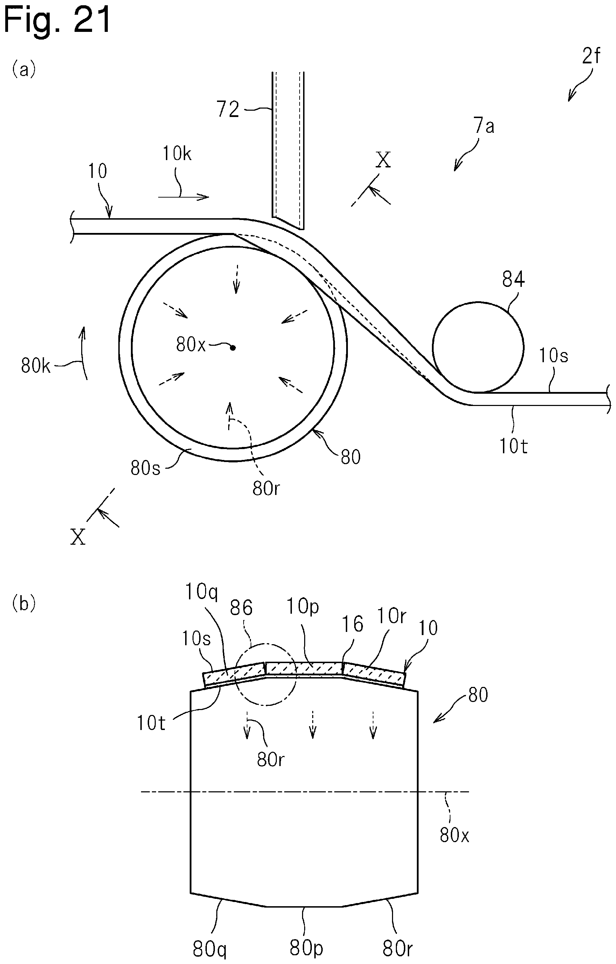

A twelfth modification in which the particulate matter is dispersed with the slits of the main sheet being widened will be described with reference to Figs. 21A to 23.

Fig. 21A is a schematic view showing a relevant part structure of an absorbent body manufacturing device 2f. As shown in Fig. 21A, a dispersing device 7a that the absorbent body manufacturing device 2f is provided with is structured such that an outer peripheral surface 80s of a substantially barrel-shaped drum 80 supports the second main surface 10t of the part, where the slits will be formed, of the main sheet 10 so as to convexly warp the first main surface 10s of the main sheet 10 on a cross section vertical to the conveying direction, and then the particulate matter supply device 72 disperses the particulate matter under a condition where the slits are widened, and then a guide roll 84 returns the main sheet 10 to a flat state.

The drum 80 rotates about a rotation center line 80k in the direction shown by the arrow 80k in such a way as to synchronize with the conveyance of the main sheet 10 in the direction shown by the arrow 10k. On the outer peripheral surface 80s of the drum 80, a plurality of non-illustrated vacuum holes are formed, and by performing air suction toward the inside of the drum 80 as shown by the arrow 80r, the second main surface 10t of the main sheet 10 is sucked and held on the outer peripheral surface 80s of the drum 80.

Fig. 21B is a schematic cross-sectional view of Fig. 21A taken along line X-X, and a detailed illustration of the cross section of the drum 80 is omitted. As shown in Fig. 21B, the outer peripheral surface 80s of the drum 80 is tapered so as to bend at positions corresponding to the slits 16 of the main sheet 10.

While the number of slits 16 formed in the main sheet 10 may be one, or three or more, for example, when two slits 16 are formed in the main sheet 10, the outer peripheral surface 80s of the drum 80 is sectioned into a cylindrical central region 80p and conical outer regions 80q and 80r on both sides of the central region 80p. The outer regions 80q and 80r are large in diameter on the side of the central region 80p and small in diameter on the side opposite to the central region 80p. The central region 80p of the outer peripheral surface 80s of the drum 80 supports the central region 10p between the two slits 16 of the main sheet 10, and the outer regions 80q and 80r of the outer peripheral surface 80s of the drum 80 support the outer regions 10q and 10r on both sides of the central region 10p of the main sheet 10. Thereby, the main sheet 10 bends at positions corresponding to the slits 16, so that the slits 16 are widened.

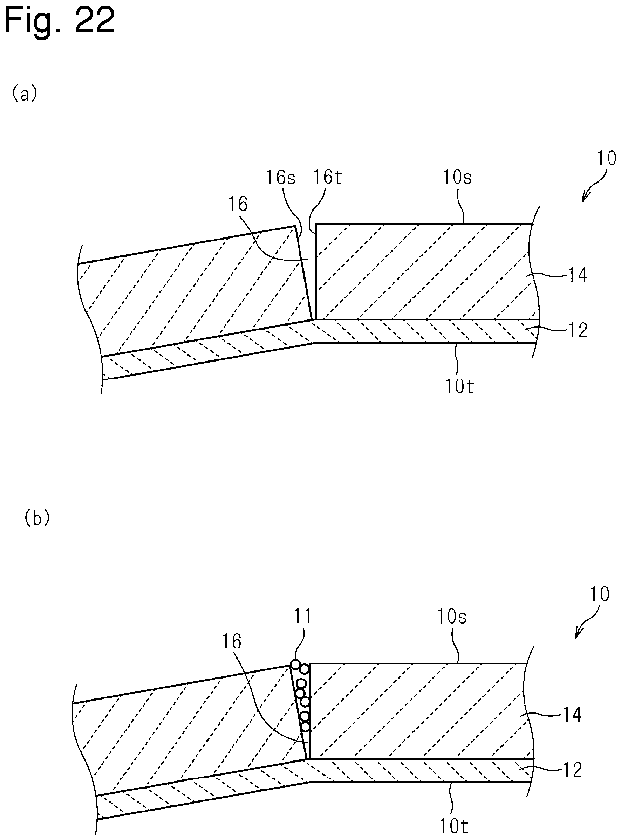

Figs. 22A and 22B are enlarged cross-sectional views of the main sheet 10 corresponding to the part surround by the chain line 86 in Fig. 21B. As shown in Fig. 22A, the main sheet 10 bends at the position corresponding to the slit 16, so that the slit 16 is widened. That is, the interval between the cut surfaces 16s and 16t increases. If the particulate matter 11 is dispersed with the slits 16 being widened like this, as shown in Fig. 2B, the particulate matter 11 readily enters the slits 16. When the particulate matter 11 is dispersed with the slits 16 being widened, compared with when the particulate matter 11 is dispersed without the slits 16 being widened, the amount of particulate matter 11 entering the slits 16 can be increased, so that the amount of particulate matter 11 entering the inside of the non-woven fabric sheet 14 of the main sheet 10 from the slits 16 can be increased.

Fig. 23 is a schematic cross-sectional view similar to Fig. 21B. As shown in Fig. 23, an outer peripheral surface 80t of a drum 80a may be roundly warped. In this case, by the second main surface 10t of the main sheet 10 being supported by the outer peripheral surface 80t of the drum 80, on the cross section of the main sheet 10 in the width direction vertical to the conveying direction, the main sheet 10s side of the main sheet 10 is convexly warped, so that the slits 16 are widened. For this reason, similarly to the drum 80 with the tapered outer peripheral surface 80s, the amount of particulate matter entering the slits 16 can be increased, so that the amount of particulate matter entering the inside of the non-woven fabric sheet of the main sheet 10 from the slits 16 can be increased.

### <Summary>

As described above, according to the present invention, an absorbent body with which it is easy to enhance liquid permeability and liquid diffusibility can be manufactured.

The present invention is not limited to the above-described embodiment but may be carried out with various modifications being added.

For example, a structure that is an appropriate combination of the structures of the first embodiment and the first to twelfth modifications may be adopted. For example, an absorbent body may be manufactured as in the tenth modification with the support sheet 12 in the first modification being replaced by a covering sheet. By appropriately combining the fifth to seventh modifications, a plurality of slits between which at least two of the length, the depth and the orientation are different may be formed on the bulky structure of the non-woven fabric sheet of the main sheet.

One or more of the above-described dispersing device, slit forming device and fiber raising device that forms the bulky structure may be disposed more than one in number in the conveying direction of the main sheet. For example, when the slits 16m and 16n that are different in inclination angle are formed as shown in Fig. 14C, a slit forming device that forms the slits 16m having one inclination angle and another slit forming device that forms the slits 16n having the other inclination angle are disposed side by side in the conveying direction of the main sheet, whereby the slits 16m and 16n that are different in inclination angle can be easily formed. Moreover, a slit forming device that forms slits in the longitudinal direction of the main sheet and another slit forming device that forms slits in the width direction of the main sheet are disposed side by side in the conveying direction of the main sheet, whereby slits intersecting each other can be formed. Moreover, by disposing a plurality of fiber raising devices in the conveying direction of the main sheet, a bulkier non-woven fabric can be formed. Moreover, by disposing a plurality of dispersing devices in the conveying direction of the main sheet, the dispersion amount of the particulate matter can be increased. These plurality of devices may be combined together.

Moreover, the particulate matter may be made to be held in the bulky structure of the non-woven fabric sheet by also using suction or by dispersing the particulate matter by an appropriate method such as spraying the particulate matter.

### [Explanation of Reference Numerals]

2, 2a, 2b, 2c, 2d, 2e, 2f Absorbent body manufacturing device
3a First position
3b Second position
3c Third position
3d Fourth position
4 Conveying device
5 Fiber raising device
6, 6a, 6b, 6c Slit forming device
7, 7a Dispersing device
8 Covering device
10, 10a, 10b, 10p, 10q, 10r Main sheet
10s First main surface
10t Second main surface
11 Particulate matter
14 Non-woven fabric sheet
14k Bulky structure
15 Dispersion region
16, 16i, 16j, 16m, 16n Slit
17 Incision
18, 18p, 18q, 18r Covering sheet
20, 20a, 20b, 20c, 20p, 20q, 20r, 20w Absorbent body
60 Tool
60k Single-edged blade (blade)
61, 61a Tool
61k Round blade (blade)
61n Blade

## Claims

1. A method for manufacturing an absorbent body, comprising:
a first step of conveying a belt-like main sheet in a longitudinal direction of the main sheet along a predetermined conveying path, the main sheet having a first and second main surfaces, the main sheet including a non-woven fabric sheet that is made of a non-woven fabric and constitutes at least part of the first main surface;
a second step of forming, at a first position on the conveying path, a slit on the non-woven fabric sheet of the main sheet being conveyed so as to form an incision in the first main surface;
a third step of dispersing, at a second position on the conveying path on a downstream side of the first position in a conveying direction of the main sheet, particulate matter capable of absorbing and retaining liquid into a region, including the incision, of the first main surface of the main sheet being conveyed, so as to cause the particulate matter to be held in the non-woven fabric sheet; and
a fourth step of overlaying, at a third position on the conveying path on the downstream side of the second position in the conveying direction of the main sheet, a covering sheet on the region, in which the particulate matter has been dispersed, of the first main surface of the main sheet being conveyed, the covering sheet preventing passage of the particulate matter,
wherein at the second position, the non-woven fabric sheet of the main sheet includes a bulky structure constituting at least part of the first main surface of the main sheet and having a void allowing the particulate matter to enter.

2. The method for manufacturing the absorbent body according to claim 1, wherein at the second step, the slit is formed so as to cause the slit to extend in the longitudinal direction of the main sheet.

3. The method for manufacturing the absorbent body according to claim 1 or 2, wherein at the second step, the slit is formed only from the first main surface up to in front of the second main surface of the main sheet.

4. The method for manufacturing the absorbent body according to any one of claims 1 to 3, wherein at the second step, when perspectively viewed vertically to the first main surface of the main sheet, the slit is formed only in the region, of the first main surface of the main sheet, in which the particulate matter is to be dispersed at the third step.

5. The method for manufacturing the absorbent body according to any one of claims 1 to 4, further comprising:
a fifth step of forming, at a fourth position on the conveying path on an upstream side of the second position in the conveying direction of the main sheet, the bulky structure on the non-woven fabric sheet of the main sheet being conveyed,
wherein at the third step, the particulate matter enters the void of the bulky structure formed at the fifth step and is held therein.

6. A device for manufacturing an absorbent body, comprising:
a conveying device that conveys a belt-like main sheet in a longitudinal direction of the main sheet along a predetermined conveying path, the main sheet having a first and second main surfaces, the main sheet including a non-woven fabric sheet that is made of a non-woven fabric and constitutes at least part of the first main surface;
a slit forming device that is disposed so as to adjoin the first position on the conveying path and forms a slit on the non-woven fabric sheet of the main sheet being conveyed by the conveying device so as to form an incision in the first main surface;
a dispersing device that is disposed so as to adjoin a second position on the conveying path on a downstream side of the first position in a conveying direction of the main sheet and disperses particulate matter capable of absorbing and retaining liquid into a region, including the incision, of the first main surface of the main sheet being conveyed by the conveying device, so as to cause the particulate matter to be held in the non-woven fabric sheet; and
a covering device that is disposed so as to adjoin a third position on the conveying path on the downstream side of the second position in the conveying direction of the main sheet and overlays a covering sheet on the region, in which the particulate matter has been dispersed, of the first main surface of the main sheet being conveyed by the conveying device, the covering sheet preventing passage of the particulate matter,
wherein at the second position, the non-woven fabric sheet of the main sheet includes a bulky structure constituting at least part of the first main surface of the main sheet and having a void allowing the particulate matter to enter.

7. The device for manufacturing the absorbent body according to claim 6, further comprising:
a tool having a blade that is disposed so as to enter the conveying path at the first position on the conveying path from the first main surface side of the main sheet being conveyed along the conveying path, and that forms the slit on the non-woven fabric sheet of the main sheet being conveyed along the conveying path so as to cause the slit to extend in the longitudinal direction of the main sheet.

8. The device for manufacturing the absorbent body according to claim 6, further comprising:
a tool having a blade that is disposed so as to enter the conveying path at the first position on the conveying path from the first main surface side of the main sheet being conveyed along the conveying path only up to in front of the second main surface of the main sheet being conveyed along the conveying path,
thereby the blade forms the slit only from the first main surface up to in front of the second main surface of the main sheet being conveyed along the conveying path.

9. The device for manufacturing the absorbent body according to any one of claims 6 to 8, wherein when perspectively viewed vertically to the first main surface of the main sheet, the slit forming device forms the slit only in the region, of the first main surface of the main sheet, in which the particulate matter is to be dispersed by the dispersing device.

10. The device for manufacturing the absorbent body according to any one of claims 6 to 9, further comprising:
a fiber raising device that is disposed so as to adjoin a fourth position on the conveying path on an upstream side of the second position in the conveying direction of the main sheet and forms the bulky structure on the non-woven fabric sheet of the main sheet being conveyed by the conveying device,
wherein the particulate matter dispersed by the dispersing device enters the void of the bulky structure formed by the fiber raising device and is held therein.

11. An absorbent body, comprising:
a main sheet having a first and second main surfaces, the main sheet including a non-woven fabric sheet that is made of a non-woven fabric and constitutes at least part of the first main surface, wherein an incision is formed in the first main surface and a slit communicating with the incision is formed in the non-woven fabric sheet;
particulate matter that is held in a void between fibers of the non-woven fabric sheet and is capable of absorbing and retaining liquid; and
a covering sheet that is overlaid on the first main surface and prevents passage of the particulate matter.

12. The absorbent body according to claim 11, wherein the slit is formed only from the first main surface up to in front of the second main surface of the main sheet.

13. The absorbent body according to claim 11 or 12,
wherein when perspectively viewed vertically to the first main surface of the main surface, the slit is formed only in a dispersion region in which the particulate matter has been dispersed.
